# EUROPÄISCHE PATENTSCHRIFT

(11) **EP 2 368 539 B1**
(45) Veröffentlichungstag und Bekanntmachung des Hinweises auf die Patenterteilung: **05.09.2018**
(21) Anmeldenummer: 10170137.3
(22) Anmeldetag: 20.07.2010
(51) Int. Cl.: A61K 6/04

(54) **Dentallegierung**
Dental alloy
Alliage dentaire

(30) Priorität: 26.03.2010 DE 102010016171
(43) Veröffentlichungstag der Anmeldung: 28.09.2011
(73) Patentinhaber: DENTSPLY SIRONA Inc., York, PA 17401-2991 (US)
(72) Erfinder: Hachenberg, Jörg, Dr., 63739 Aschaffenburg (DE); Steinke, Rudi, 63457 Hanau (DE); Klaus, Angela, 63456 Hanau (DE); Wissel, Irmgard, 63457 Freigericht (DE)
(74) Vertreter: Stoffregen, Hans-Herbert

(56) Entgegenhaltungen:
- EP-A1- 1 340 482
- DE-A1- 3 132 143
- DE-A1- 3 830 666
- US-A- 4 518 564
- US-A- 5 462 437
- S.M. Dunne et al.: "A study into the performance of gallium-based restorative material", British Dental Journal, vol. 189, no. 6 6 September 2000 (2000-09-06), page 306, Retrieved from the Internet: URL:http://www.nature.com/bdj/journal/v189 /n6/pdf/4800752a.pdf [retrieved on 2014-11-24]

## Beschreibung

Die Erfindung bezieht sich auf eine Palladium-dominierte Dentallegierung, insbesondere aufbrennfähige Dentallegierung zum Herstellen von Zahnersatz, wie Kronen, Brücken, Inlays oder Onlays, enthaltend zumindest Gold, Palladium und Silber sowie einen Kornfeiner in Form von Ruthenium.

Eine Dentallegierung gemäß der DE-C-32 11 703 enthält in Gew.-% Gold 10 - 60 %, Palladium 20 - 60 %, Silber 0 - 15 %. Ferner sind 0 - 10 % Indium, 0 - 10 % Zinn, 0 - 5 % Zink, 0 - 2 % Iridium, 0 - 2 % Kupfer, 0,1 - 5 % Platin und/oder jeweils 0,05 - 2 % von mindestens einem der Übergangsmetalle der 4., 5. und 6. Nebengruppe des Periodensystems enthalten.

Um eine hohe mechanische Stabilität, insbesondere eine reproduzierbare Hochtemperaturstabilität bei Palladium- und Kupfer-freien hochgoldhaltigen Dentallegierungen zu erzielen, ist es nach der EP-B-1 799 873 bekannt, der Legierung einen einzigen Kornfeiner und spezifische weitere Elemente hinzuzufügen, die in Kombination zu einer Verminderung von unerwünschten Kornfeinungsagglomeraten führen. Bei dem Kornfeiner handelt es sich um Iridium oder Rhodium, wobei bei Vorliegen von Niob in der Legierung der Kornfeiner Iridium, bei Vorliegen von Tantal der Kornfeiner Rhodium und bei Vorliegen von Titan oder Vanadium der Kornfeiner Iridium oder Rhodium ist.

Auch sind so genannte goldreduzierte Palladiumlegierungen bekannt, in denen der Palladiumanteil in Atom-% stets größer als der Gold-Anteil ist, so dass von Palladium-dominierten Legierungen gesprochen wird, gleichwenn der Gew.-%-Anteil von Gold größer als der von Palladium sein kann. Bei entsprechenden Palladium-dominierten Legierungen kann Ruthenium als Kornfeiner enthalten sein. Allerdings hat sich herausgestellt, dass Ruthenium zu keiner gesteuerten Kornfeinung führt.

Eine goldarme Dental-Edelmetalllegierung zum Aufbrennen von Porzellan nach der DE-B-28 28 304 enthält neben Gold, Palladium und Silber Titan mit einem Anteil zwischen 0,05 und 0,5 %.

Der DE-C-29 44 755 ist eine zahnärztliche Legierung zum Aufbrennen auf Porzellan zu entnehmen, in der Ruthenium mit einem Anteil zwischen 0,175 und 0,25 Gew.% und Silber mit einem Anteil zwischen 5,86 und 11 Gew.% enthalten sind.

Eine Zahnlegierung nach der EP-A-0 057 149 enthält bis zu 20 Gew.% Silber, bis 40 Gew.% Palladium, bis 20 Gew.% Platin und bis 30 Gew.% Gold. Ferner können bis zu 3 Gew.% Ruthenium, Iridium und Rhodium bei einem Anteil von Zink bis zu 5 Gew.% und Kupfer bis 8 Gew.% enthalten sein. In der Legierung selbst ist weder Iridium noch Zinn enthalten. Allergene Metalle wie Eisen, Cobalt, Nickel, Chrom und Mangan sind mit einem Anteil von weniger als 0,05 Gew.% enthalten.

Eine Edelmetalllegierung nach der DE-A-31 46 794 enthält zwingend Gallium.

Bei einer goldfarbenen Palladium-Indium-Legierung nach der WO-A-90/07018 beträgt der Goldanteil maximal 30 Gew.%.

Eine Dentallegierung nach der US-A-2002/0122741 enthält 15 - 75 % Ag, bis 55 % Au, 10 - 50 % Pd, 6 - 25 % Pt sowie gegebenenfalls In, Ga, Sn, Ge, Zn, Mn, Ir, Ru, Rh, Re, wobei der Anteil von Ir, Ru, Rh, Re jeweils für sich und in Kombination maximal 3 % beträgt.

Palladium-Basislegierungen nach der DE-A-38 30 666 enthalten Gold bis zu 20 Gew.% sowie Gallium zwischen 0,5 und 5 Gew.%.

Der US-A-4 518 564 A ist eine Gallium- und Silber-freie Palladium-dominierte Legierung zu entnehmen.

In der Veröffentlichung S.M. Dunne et al.: "A study into the performance of galliumbased restorative material", British Dental Journal, Bd. 189, Nr. 6 (2000-09-06), Seite 306, werden Untersuchungen von Dentallegierungen beschrieben, die Gallium enthalten.

Der vorliegenden Erfindung liegt die Aufgabe zu Grunde, eine Palladium-dominierte Legierung der eingangs genannten Art so weiterzubilden, um eine feinkörnige Ausscheidung unter Vermeidung von Agglomeraten zur Erzielung einer Dentallegierung hoher mechanischer Stabilität sowie sehr guter Polierbarkeit zur Verfügung stellen zu können.

Zur Lösung der Aufgabe wird im Wesentlichen vorgeschlagen, dass die Dentallegierung in Gew.% besteht aus:

| | |
|---|---|
| 38 - 42 | Gold |
| 38 - 42 | Palladium |
| 12 - 14 | Silber |
| 6 - 9 | Zinn |
| 0,05 - 1,0 | Ruthenium |
| 0,05 - 2,0 | Kornfeinungssteuerungselement, |

wobei zumindest ein Element aus der Gruppe Tantal, Niob, Yttrium, Zirkonium, Chrom, Molybdän das Kornfeinungssteuerungselement ist, und
wobei die Gesamtsumme der Elemente 100 Gew.% beträgt.

Durch den Zusatz zumindest eines Kornfeinungssteuerungselementes wird erfindungsgemäß erreicht, dass dieses mit dem Kornfeiner wie Ruthenium ein Phasendiagramm besitzt, welches ein Eutektikum aufweist.

Dadurch wird die flüssige Phase, welche den Kornfeiner wie Ruthenium, das zumindest eine Kornfeinungssteuerungselement und die restlichen Bestandteile der Legierung enthält, stabilisiert. Der Kornfeiner wie Ruthenium scheidet sich im Vergleich zur Erstarrung unter Abwesenheit eines Kornfeinungssteuerungselements erst bei einer niedrigeren Temperatur und feiner verteilt mit dem zumindest einen Kornfeinungssteuerungselement aus. Diese Ausscheidungen wirken bei der Erstarrung der übrigen Bestandteile als Kristallisationskeime, so dass insgesamt eine feinere Gefügestruktur erzielbar ist.

Bevorzugterweise ist das Kornfeinsteuerungselement Tantal. Auch Niob oder eine Mischung aus Niob und Tantal sind hervorzuheben.

Der Zinn-Anteil dient der Steigerung der mechanischen Festigkeit der Legierung. Dies wird bekanntermaßen durch Zugabe von unedlen Komponenten wie Zinn, Zink, Indium und Kupfer erreicht, wobei aber Zinn im bevorzugten Zusammensetzungsbereich zu den besten Ergebnissen geführt hat. Auch Platin führt in gewissem Umfang zu einer Festigkeitssteigerung, ist jedoch sehr teuer.

Unabhängig davon wird eine Härtesteigerung auch durch Zusatz von Gallium erreicht, wobei Gallium die Biokompatibilität negativ beeinflussen kann. Die Legierung ist daher bevorzugt Gallium-arm oder -frei.

Insbesondere zeichnet sich die Erfindung dadurch aus, dass die Dentallegierung in Gew.% besteht aus

| | |
|---|---|
| 39,0 - 41, 0 | Gold |
| 39,0 - 41, 0 | Palladium |
| 12,0 - 13,0 | Silber |
| 6,5 - 8,0 | Zinn |
| 0,05 - 0,5 | Ruthenium und |
| 0,1 - 1,0 | Tantal oder |
| 0,1 - 1,0 | Niob. |

Die Gesamtsumme der Elemente beträgt wiederum genau 100 Gew.%.

Eine bevorzugte Zusammensetzung der Dentallegierung in Gew.-% zeichnet sich aus durch 40,0 Au, 39,8 Pd, 12,4 Ag, 7,5 Sn, 0,2 Ta, 0,1 Ru.

Erfindungsgemäß wird eine Palladium-dominierte Legierung, bei der die chemischen und metallphysikalischen Eigenschaften von Palladium bestimmt werden, vorgeschlagen, die im Vergleich zum Stand der Technik eine feinere Gefügestruktur unter Vermeidung von Agglomeraten aufweist, die ansonsten die mechanische Stabilität sowie die Polierbarkeit negativ beeinflussen.

Hierzu werden ein Kornfeiner und zumindest ein Kornfeinungssteuerungselement als Bestandteile der Dentallegierung hinzugefügt, wobei der Kornfeiner und das zumindest eine Kornfeinungssteuerungselement ein Phasendiagramm besitzen, das ein Eutektikum aufweist.

Die Temperatur des Eutektikums im binären Phasendiagramm des Kornfeiners Ruthenium mit dem Kornfeinungssteuerungselement liegt dabei bevorzugterweise mehr als 250 K unterhalb des tieferen Erstarrungspunktes der reinen Elemente, da ein tiefes Eutektikum hier wünschenswert ist. Naturgemäß muss es oberhalb der Schmelztemperatur der Dentallegierung liegen. Somit scheiden sich zunächst der Kornfeiner und das zumindest eine Kornfeinungssteuerungselement aus, die Kristallationskeime bilden, ohne jedoch genug Zeit zur Agglomeration zu besitzen, was folglich die gewünschte feine Gefügestruktur ermöglicht.

In der nachfolgenden Tabelle werden bekannte Legierungen bzw. Legierungen, die nicht die erfindungsgemäße Zusammensetzung aufweisen, und Legierungen nach der erfindungsgemäßen Lehre aufgelistet. Anhand von Schliffbildern konnte festgestellt werden, dass Dentallegierungen, die Ruthenium als Kornfeiner und Tantal bzw. Niob als Kornfeinungssteuerungselement enthalten, feinkörnig sind, wobei die die Stabilität und Polierbarkeit negativ beeinflussenden Agglomerate nicht oder nur im vernachlässigten Umfang auftreten.

| Legierung | Zusammensetzung in gew% | | | | | | | | | | | | Ergebnis |
|---|---|---|---|---|---|---|---|---|---|---|---|---|---|
| | Pd | Au | Ag | Sn | In | Zn | Ga | Pt | Ru | Ta | Nb | Ir | |
| Bekannte Legierung I | 35,00 | 39,00 | 19,40 | 5,00 | 0,50 | - | - | 1,00 | 0,05 | - | - | 0,05 | Grobkörnig, stark geseigert, ausgeprägte Agglomerate |
| Bekannte Legierung II | 39,40 | 40,00 | 10,00 | 0,20 | 8,80 | - | 1,40 | - | 0,20 | - | - | - | Grobkörnig, 2. Phase (Ga-reich), ausgeprägte Agglomerate |
| Bekannte Legierung III | 35,60 | 40,00 | 17,60 | 5,00 | - | - | 0,50 | 1,00 | - | 0,10 | - | 0,20 | Grobkörnig, stark geseigert, 2. Phase (Ga-reich), einige Agglomerate |
| EHF10* | 39,80 | 40,00 | 12,02 | 8,00 | - | - | - | - | 0,09 | - | - | 0,09 | Relativ Feinkörnig, aber stark ausgeprägte Agglomerate |
| EHF12* | 39,80 | 40,00 | 13,10 | 7,00 | - | - | - | - | 0,10 | - | - | - | Dendritisch/Grobkör nig einige Agglomerate |
| EHF13 | 39,80 | 40,00 | 12,80 | 7,00 | - | - | - | - | 0,10 | - | 0,3 0 | - | Feinkörnig, nahezu keine Agglomerate |
| EHF14 | 39,80 | 40,00 | 12,90 | 7,00 | - | - | - | - | 0,10 | 0,20 | - keine | - | Feinkörnig, nahezu keine Agglomerate |
| EHF16 | 39,80 | 40,00 | 12,40 | 7,50 | - | - | - | - | 0,10 | 0,20 | - | - | Feinkörnig, nahezu keine Agglomerate |
| EHF19 * | 50,00 | 20,00 | 25,00 | - | 4,70 | - | - | - | 0,10 | 0,20 | - | - | Feinkörnig, keine Agglomerate |

| | | | | | | | | | | | | | |
|---|---|---|---|---|---|---|---|---|---|---|---|---|---|
| * nicht erfindungsgemäße Versuchsbeispiele | | | | | | | | | | | | | |

## Patentansprüche

1. Palladium-dominierte Dentallegierung, insbesondere aufbrennfähige Dentallegierung zum Herstellen von Zahnersatz, wie Kronen, Brücken, Inlays oder Onlays, enthaltend zumindest Gold, Palladium und Silber, sowie einen Kornfeiner in Form von Ruthenium,
**dadurch gekennzeichnet,**
**dass** die Dentallegierung in Gew.% besteht aus
| | |
|---|---|
| 38 - 42 | Gold |
| 38 - 42 | Palladium |
| 12 - 14 | Silber |
| 6 - 9 | Zinn |
| 0,05 - 1,0 | Ruthenium |
| 0,05 - 2,0 | Kornfeinungssteuerungselement, |
wobei zumindest ein Element aus der Gruppe Tantal, Niob, Yttrium, Zirkonium, Chrom, Molybdän das Kornfeinungssteuerungselement ist, und
wobei die Gesamtsumme der Elemente 100 Gew.% beträgt.

2. Palladium-dominierte Dentallegierung nach Anspruch 1,
**dadurch gekennzeichnet,**
**dass** die Dentallegierung in Gew.% besteht aus
| | |
|---|---|
| 39,0 - 41, 0 | Gold |
| 39,0 - 41, 0 | Palladium |
| 12,0 - 13,0 | Silber |
| 6,5 - 8,0 | Zinn |
| 0,05 - 0,5 | Ruthenium und |
| 0,1 - 1,0 | Tantal oder |
| 0,1 - 1,0 | Niob. |

3. Palladium-dominierte Dentallegierung nach Anspruch 1,
**dadurch gekennzeichnet,**
**dass** die Dentallegierung in Gew. % besteht aus:
| | |
|---|---|
| 40,0 | Gold |
| 39,8 | Palladium |
| 12,4 | Silber |
| 7,5 | Zinn |
| 0,2 | Tantal |
| 0,1 | Ruthenium |

4. Verwendung der Palladium-dominierten Dentallegierung nach zumindest Anspruch 1 als aufbrennfähige Dentalllegierung zum Herstellen von Zahnersatz, wie Kronen, Brücken, Inlays oder Onlays.

## Claims

1. Palladium-dominated dental alloy, in particular a directly-bondable dental alloy for the production of a dental prosthesis, such as crowns, bridges, inlays or onlays, containing at least gold, palladium and silver, as well as a grain refiner in the form of ruthenium,
**characterised in that**
the dental alloy has the following constituents, in percentage by weight:
| | |
|---|---|
| 38 - 42 | Gold |
| 38 - 42 | Palladium |
| 12 - 14 | Silver |
| 6-9 | Tin |
| 0.05 - 1.0 | Ruthenium |
| 0.05 - 2.0 | Grain-refining control element, |
wherein the grain-refining element is at least one element from the group consisting of tantalum, niobium, yttrium, zirconium, chromium, molybdenum, and wherein the total sum of the elements is 100% by weight.

2. Palladium-dominated dental alloy according to claim 1,
**characterised in that**
the dental alloy has the following constituents in percentage by weight:
| | |
|---|---|
| 39.0 - 41.0 | Gold |
| 39.0 - 41.0 | Palladium |
| 12.0 - 13.0 | Silver |
| 6.5 - 8.0 | Tin |
| 0.05 - 0.5 | Ruthenium and |
| 0.1 - 1.0 | Tantalum or |
| 0.1 - 1.0 | Niobium. |

3. Dental alloy according to claim 1,
**characterised in that**
the dental alloy has the following constituents in percentage by weight:
| | |
|---|---|
| 40.0 | Gold |
| 39.8 | Palladium |
| 12.4 | Silver |
| | |
|---|---|
| 7.5 | Tin |
| 0.2 | Tantalum |
| 0.1 | Ruthenium. |

4. Use of the palladium-dominated dental alloy according to at least claim 1 as a directly-bondable dental alloy for the production of a dental prosthesis such as crowns, bridges, inlays or onlays.

## Revendications

1. Alliage dentaire dominé par le palladium, en particulier alliage dentaire céramo-métallique pour la fabrication de prothèses dentaires, telles que des couronnes, des bridges, des inlays ou des onlays, contenant au moins de l'or, du palladium et de l'argent, ainsi qu'un affineur de grain sous forme de ruthénium,
**caractérisé en ce**
**que** l'alliage dentaire est composé en % pondéral de
| | |
|---|---|
| 38 - 42 | or |
| 38 - 42 | palladium |
| 12 - 14 | argent |
| 6 - 9 | étain |
| 0,05 - 1,0 | ruthénium |
| 0,05 - 2,0 | élément de contrôle de l'affinage du grain, |
sachant qu'au moins un élément du groupe tantale, niobium, yttrium, zirconium, chrome, molybdène est l'élément de contrôle de l'affinage du grain, et que la somme totale des éléments est de 100 % en valeur pondérale.

2. Alliage dentaire dominé par le palladium selon la revendication 1,
**caractérisé en ce**
**que** l'alliage dentaire est composé en % pondéral de
| | |
|---|---|
| 39,0 - 41,0 | or |
| 39,0 - 41,0 | palladium |
| 12,0 - 13,0 | argent |
| 6,5 - 8,0 | étain |
| 0,05 - 0,5 | ruthénium et |
| 0,1 - 1,0 | tantale ou |
| 0,1 - 1,0 | niobium. |

3. Alliage dentaire dominé par le palladium selon la revendication 1,
**caractérisé en ce**
**que** l'alliage dentaire est composé en % pondéral de:
| | |
|---|---|
| 40,0 | or |
| 39,8 | palladium |
| | |
|---|---|
| 12,4 | argent |
| 7,5 | étain |
| 0,2 | tantale |
| 0,1 | ruthénium. |

4. Utilisation de l'alliage dentaire dominé par le palladium selon au moins la revendication 1 comme alliage dentaire céramo-métallique pour la fabrication de prothèses dentaires, telles que des couronnes, des bridges, des inlays ou des onlays.
